# EUROPÄISCHE PATENTSCHRIFT

(11) **EP 1 378 206 B1**
(45) Veröffentlichungstag und Bekanntmachung des Hinweises auf die Patenterteilung: **21.01.2004**
(21) Anmeldenummer: 02020474.9
(22) Anmeldetag: 12.09.2002
(51) Int. Cl.: A61B 19/00

(54) **Röntgenbildunterstützte Navigation mit ursprünglichen, zweidimensionalen Röntgenbildern**
X-ray supported navigation with original, two-dimensional x-ray images
Navigation à l'aide de rayons X avec des images radiographiques intitialles à deux dimensions

(43) Veröffentlichungstag der Anmeldung: 07.01.2004
(73) Patentinhaber: BrainLAB AG, 85551 Kirchheim/Heimstetten (DE); Siemens AG, 80333 München (DE)
(72) Erfinder: Essenreiter, Robert, 81825 München (DE); Seifferth, Falko, 85604 Zorneding (DE); Schmidt, Robert, 85551 Kirchheim (DE); Zeiss, Mario, 85586 Poing (DE); Neagu, Sorin-Alexandru, 91052 Erlangen (DE); Mühlhäusser, Matthias, 91074 Herzogenaurach (DE); Steiner, Andres, 90491 Nürnberg (DE)
(74) Vertreter: Schwabe - Sandmair - Marx

(56) Entgegenhaltungen:
- DE-A- 19 807 884
- DE-A- 19 917 867
- US-A- 5 222 499
- US-B1- 6 351 573

## Beschreibung

Die vorliegende Erfindung betrifft die röntgenbildunterstützte Navigation im medizinischen Bereich. Sie stellt ein Verfahren zur Bilddarstellung bei einem medizinischen Navigationssystem mit Röntgenbildunterstützung zur Verfügung.

Die moderne, computergestützte Chirurgie ist mit Hilfe von medizinischen Navigationssystemen in der Lage, während der Operation Instrumente oder Implantate des Chirurgen auf einem Monitor in Relation zu anatomischen Bilddaten darzustellen. Diese anatomischen Bilddaten eines Patienten können entweder prä- oder aber intra-operativ akquiriert werden und aus verschiedenen Modalitäten bestehen (CT, MRI, PET, SPECT, Röntgenbilder oder Kombinationen hiervon).

In der computergestützten Chirurgie ist es derzeit auch üblich, mit Hilfe eines Röntgengerätes, das meist in Form eines C-Bogens verwendet wird, Bilddaten zu erheben und diese Daten dann mit Hilfe eines Navigationssystems nutzbar zu machen. Das Computersystem verarbeitet die Bilddaten und "registriert" sie, d. h. das System bestimmt die genaue Position von Bilddaten in Relation zu einer vorher definierten Referenz, zum Beispiel am Patienten im Raum. Es ist danach möglich, auf diesen gewonnenen Daten die Position eines Instruments darzustellen, d. h. zu navigieren.

Die röntgenbasierte Navigation hat den Vorteil, dass sie es durch ein sehr einfaches Registrierungsverfahren dem Chirurgen ermöglicht, einfach, unkompliziert und schnell die Hilfe der Navigation in Anspruch nehmen zu können. Isoliert stellt die Röntgennavigation eine wertvolle Hilfe für den Chirurgen zur Verfügung, jedoch hat sie bei den derzeit angewandten Methoden den Nachteil, dass jedes Bild einzeln registriert und dann in richtiger Relation zu den Instrumenten oder Apparaten dargestellt werden muss.

Ferner existieren Systeme, die sowohl die Navigation auf der Basis von Volumendatensätzen (zum Beispiel CT, MRI) nutzen, als auch zusätzlich einzeln zu registrierende Röntgenbilder bzw. rekonstruierte Röntgenbilder, die aus dem Volumendatensatz bzw. aus verarbeiteten Röntgenbildsätzen heraus rekonstruiert werden. Der Nachteil hierbei besteht darin, dass die Röntgenbilder im ursprünglichen Zustand eigentlich sehr gute Abbildungsdaten mit hohem Kontrast und guter Detailgenauigkeit liefern, diese Vorteile jedoch bei der Umrechnung in einen 3D-Volumendatensatz sowie bei der nochmaligen Zurückrechnung auf rekonstruierte Röntgenbilder teilweise verloren gehen. Der Chirurg muss dann mit Bilddaten navigieren, deren Bildinhalt weniger gut ist als derjenige der ursprünglich akquirierten Röntgenbilder.

Aus der WO 96/11624 ist ein chirurgisches Navigationssystem mit Referenz- und Lokalisierungsrahmen bekannt. Die US 5,980,535 offenbart eine Vorrichtung für ein anatomisches Tracking-Verfahren, bei dem Positionssignale verarbeitet werden, die von einem Referenzobjekt an einem Schwing-Arm einer Kopfklammer stammen.

Die US 6,314,310 B1 offenbart ein röntgenbild-geführtes, chirurgisches Lokalisierungssystem mit erweitertem Mapping-Volumen, bei dem Röntgenbilder aus einem Fluoroskop(C-Bogen)-Röntgengerät über Referenzelemente in Relation zu chirurgischen Instrumenten registriert werden. Aus der DE 199 17 867 A1 ist ein Verfahren und eine Vorrichtung zur bildunterstützten Behandlung von Behandlungszielen mit Integration von Röntgenerfassung und Navigationssystem bekannt. Die US 6,048,097 zeigt eine Röntgen-Untersuchungsvorrichtung mit einem C-Bogen auf, wobei in mindestens einer Position des C-Armes eine isozentrische Bestrahlung erfolgt.

Es ist die Aufgabe der vorliegenden Erfindung, ein Verfahren für die röntgenbildunterstützte Navigation zur Verfügung zu stellen, welches die oben genannten Nachteile des Standes der Technik überwindet. Insbesondere soll gewährleistet werden, dass dem Chirurgen detailgenaue Röntgenbilder für die Navigation zur Verfügung gestellt werden. Ferner soll die aufwändige Einzelregistrierung intra-operativ erstellter Röntgenbilder möglichst vermieden werden.

Diese Aufgabe wird erfindungsgemäß gelöst durch ein Verfahren zur Bilddarstellung bei einem medizinischen Navigationssystem mit Röntgenbildunterstützung, bei dem mittels eines vorab im Navigationssystem kalibrierten Röntgengerätes eine Vielzahl von Röntgenbildern eines Patienten aus unterschiedlichen Positionen erstellt werden, während mittels eines medizinischen Navigationssystems eine Positionsbestimmung für das Röntgengerät erfolgt, die erhaltenen Patientendaten aus den erstellten Röntgenbildem durch Verarbeitung im Röntgengerät-System auf der Basis der Kalibrierung ausgewertet und räumlich umgesetzt werden, zusätzlich zu den ausgewerteten und umgesetzten Patientendaten noch die ursprünglichen zweidimensionalen Röntgenbilder und die den Röntgenbildern zugehörigen Erstellungsinformationen an das Navigationssystem übergeben werden, und bei dem die zweidimensionalen Röntgenbilder navigationsunterstützend auf einer Bildausgabe des Navigationssystems angezeigt werden.

Der Vorteil des erfindungsgemäßen Verfahrens liegt insbesondere in der Bereitstellung der ursprünglichen, zweidimensionalen Röntgenbilder begründet. Diese Röntgenbilder sind detailgetreuer als die herkömmlicherweise verwendeten, aus dem Volumendatensatz rekonstruierten Röntgenbilder und sie weisen einen besseren Kontrast auf. Ferner ist es derzeit für Chirurgen, die es gewohnt sind, zweidimensionale Röntgenbilder zu interpretieren, auch leichter, diese ursprünglichen Röntgenbilder in ihrer gewohnten Form auch bei der Navigation zu interpretieren. Da die vom Patienten erstellten Röntgenbilder schon gleich bei der Erstellung im Navigationssystem registriert werden und die Bilddaten zusammen mit den Registrierungsinformationen für alle Bilder zur Verfügung stehen, ist es nicht mehr notwendig, erstellte Röntgenbilder jeweils einzeln und separat zu registrieren, um die Navigation zu ermöglichen. Der Chirurg kann wahlweise auf den aus den erhaltenen Bilddaten erstellten dreidimensionalen Darstellungen (Volumendatensatz) navigieren oder auf den gewohnten zweidimensionalen, schon vorab registrierten Röntgenbildern. Natürlich kann die Navigation auch simultan in allen Bilddarstellungen erfolgen. Die zweidimensionalen, ursprünglichen Röntgenbilddaten, können besonders visualisiert werden, es ist beispielsweise möglich, dass der Chirurg diese Bilder sozusagen "durchblättert", bis er das gewünschte, aussagekräftigste Bild für die Navigation vor sich hat. Insbesondere ist auch von Vorteil, dass ohne zusätzliche Registrierung mit Hilfe der zweidimensionalen Röntgenbildern navigiert werden kann.

Zur Kalibrierung des Röntgengerätes kann dessen Position vorteilhafterweise in der Relation zu einem vom Navigationssystem erfassbaren Kalibrierungsphantom ermittelt werden. Gemäß einer bevorzugten Ausführungsform der Erfindung werden während der Kalibrierung des Röntgengerätes Transformationsmatrizen betreffend die Raumlage der aufgenommenen Röntgenbilder erstellt. Dabei können bevorzugt mit der Übergabe der ursprünglichen, zweidimensionalen Röntgenbilder an das Navigationssystem auch die den einzelnen Röntgenbildem zugeordneten Transformationsmatrizen übergeben werden.

In bevorzugter Ausgestaltung wird bei dem erfindungsgemäßen Verfahren als Röntgengerät ein C-Bogen-Röntgengerät verwendet, das Röntgenbilder in verschiedenen Drehstellungen darstellt, insbesondere isozentrische Röntgenbildfolgen.

Die Erfindung betrifft ferner ein Programm gespeichert auf ein computer-lesbares Speichermedium das, wenn es auf einem Computer läuft oder in einen Computer geladen ist, den Computer veranlasst, eines der oben genannten Verfahren durchzuführen.

Die Erfindung wird im Weiteren anhand der beiliegenden Figuren und mittels eines Ausführungsbeispiels näher erläutert. Es zeigen:
- Figur 1: ein C-Bogen-Röntgengerät, ein Kalibrierungsverfahren;
- Figur 2: das C-Bogen-Röntgengerät aus Figur 1 bei der Bildaufnahme; und
- Figur 3: eine Bilddarstellung, wie sie gemäß dem erfindungsgemäßen Verfahren bereitgestellt werden kann.

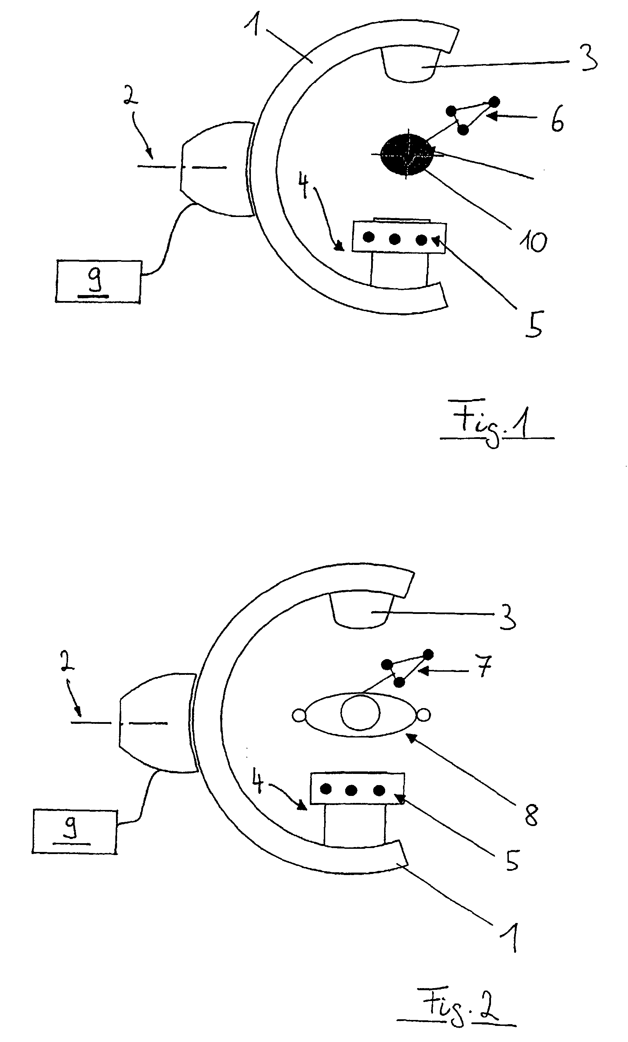

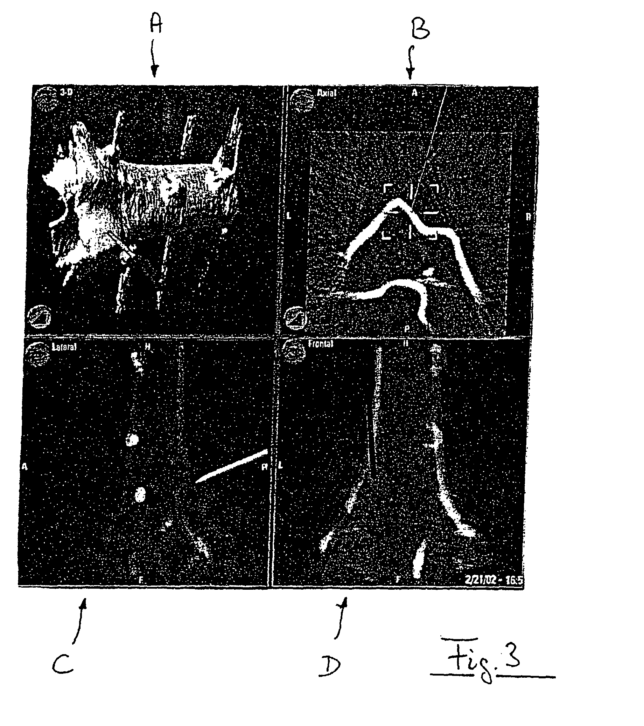

Die Figur 1 zeigt ein C-Bogen-Röntgengerät 1, dessen Bogen sich zur Aufnahme von Röntgenbildem um die Achse 2 drehen kann, so dass eine Folge isozentrischer Röntgenbildaufnahmen erstellt werden kann. Am C-Bogen 1 ist auf der Oberseite die Strahlungsquelle 3 und gegenüber der Bildaufnehmer 4 dargestellt, der einen Kalibrierungsaufsatz trägt, welcher wiederum eine Markergeometrie 5 aufweist. Schematisch ist das Navigationssystem 9 eingezeichnet, welches beispielsweise durch Kameras die Positionen des C-Bogens (über die Markergeometrie 5) und von im Bestrahlungsfeld vorhandenen Objekten (zum Beispiel Kalibrierungsphantom 10 mit Markergeometrie 6 in Figur 1 bzw. Patient 8 mit Markergeometrie 7 in Figur 2) positionell erfasst und verfolgt. Über eine nicht bezeichnete Datenverbindung können vom C-Bogen-Röntgengeräte erfasste und möglicherweise schon verarbeitete Daten an das Navigationssystem 9 weitergeleitet werden.

In Figur 1 ist das C-Bogen-Röntgengerät 1 beim Kalibrierungsvorgang dargestellt, wobei hier das Kalibrierungsphantom 10 in den Strahlengang eingesetzt wird, welches die Markergeometrie 6 aufweist, die wiederum vom Navigationssystem 9 erkannt werden kann. In Figur 2 ist das C-Bogen-Röntgengerät aus Figur 1 bei der Akquirierung von Patienten-Röntgenbildern gezeigt, wobei hier der Patient 8 im Strahlengang liegt, der wiederum die Markergeometrie 7 trägt, welche vom Navigationssystem 9 erfassbar ist.

Der Ablauf eines erfindungsgemäßen Verfahrens gemäß einem Ausführungsbeispiel, bei dem prä-kalibrierte Daten genutzt werden, um eine Navigation aus zweidimensionalen und dreidimensionalen Daten simultan zu ermöglichen, wird im Folgenden erörtert:

In einem ersten Schritt wird der zur Navigation eingesetzte C-Bogen 1 in Relation zu dem Kalibrierungsphantom 10 kalibriert. Hierzu wird die Position des C-Bogens 1 in Relation zur Position des Phantoms 10 vom Kamerasystem des Navigationssystems 9 aufgenommen. Diese Kalibrierung ermöglicht es, die während der Bildaufnahme am Patienten akquirierten zweidimensionalen Daten umzurechnen, und zwar mit Hilfe von Transformationsmatrizen in Beziehung zueinander zu setzen und für die dreidimensionale Navigation einzusetzen.

Nach der Kalibrierung kann die Datenakquirierung am Patienten 8 erfolgen (Figur 2). Dazu wird die Position des C-Bogens 1 in Relation zum Patienten 8 vom Kamerasystem des Navigationssystems 9 aufgenommen. Nach dieser Positionsbestimmung erfolgt die Aufnahme einer bestimmten Anzahl zweidimensionaler Röntgenbilder, die mit Hilfe der Kalibrierung in Relation zueinander und zum C-Bogen 1 und dadurch auch zum Patienten 8 gesetzt werden.

Nach dem Abschluss der Akquirierung der zweidimensionalen Daten werden diese mit Hilfe der durch die Kalibrierung ermittelten Transformationsmatrizen in dreidimensionale Daten umgerechnet. Diese dreidimensionalen Daten werden an das Navigationssystem 9 übergeben und können danach für die computerunterstützte Chirurgie eingesetzt werden, da sie durch die Positionsbestimmung von C-Bogen 1 in Relation zum Patienten 8 bereits registriert sind.

Beispielsweise in einem Speicher des C-Bogens bleiben aber die zweidimensionalen Röntgenbilddaten in ihrer ursprünglichen Form, d. h. mit den zugehörigen Transformationsmatrizen erhalten. Erfindungsgemäß werden nunmehr nicht nur - wie bisher üblich - die Positionsinformationen (Transformationsmatrix) der dreidimensionalen Daten an das Navigationssystem 9 übergeben, sondern es erfolgt auch eine Übergabe der zweidimensionalen Daten und der jeweils dazugehörigen einzelnen Transformationsmatrix für jedes Röntgenbild. Damit werden diese ursprünglichen Daten, die mitsamt den notwendigen Raumlage-Positionsinformationen ohnehin zur Verfügung stehen, mit ihrem ursprünglichen Bildgehalt für die Navigation nutzbar, und sie können beispielsweise so dargestellt werden, wie es in Figur 3 gezeigt ist.

Diese Figur 3 zeigt ein Beispiel für die Bildausgabe eines Navigationssystems, die vier Bilder enthält, nämlich einerseits die übliche 3D-Darstellung A, die aus dem dreidimensionalen, umgerechneten Volumendatensatz erhalten wird, und andererseits drei zweidimensionale Darstellungen B (axial), C (lateral) und D (frontal). Bei den zweidimensionalen Darstellungen handelt es sich aber hier nicht um übliche Rekonstruktionen von 2D-Bildem aus dem 3D-Datensatz, der auch für die Darstellung A verwendet wird, sondern um die ursprünglich durch den C-Bogen 1 akquirierten Röntgenbilder, die schon vorab bei der Erstellung registriert wurden und für die jede einzelne Transformationsmatrix zum Erstellungszeitpunkt im Navigationssystem vorliegt, weil sie separat mit der 2D-Darstellung übergeben worden ist. Der Chirurg kann nun während der Operation sowohl auf den ursprünglichen zweidimensionalen Röntgenbildern als auch auf der dreidimensionalen Darstellung und in einer Kombination dieser Navigationsmöglichkeiten arbeiten. Vorteile sind insbesondere die bessere Darstellungsqualität der ursprünglich zweidimensionalen Röntgenbilder im Vergleich mit den dreidimensionalen, verrechneten Daten, insbesondere was den besseren Kontrast und die bessere Detailgenauigkeit betrifft. Außerdem fällt es dem Chirurgen leichter auf solchen gewohnten, detailgenauen Röntgenbildern zu navigieren, da er schon von seiner Ausbildung her an die Interpretation solcher Bilder gewöhnt ist. Es erfolgt vorteilhafterweise eine automatische Registrierung der zweidimensionalen und der dreidimensionalen Daten und es sind besondere Visualisierungsarten möglich, zum Beispiel können die Röntgenbilder B, C und D durch die Röntgenbildfolge sozusagen "durchgeblättert" werden; es kann also solange das nächste Bild aufgerufen werden, bis der Chirurg das optimale Bild für die Navigation gefunden hat.

## Patentansprüche

1. Verfahren zur Bilddarstellung bei einem medizinischen Navigationssystem mit Röntgenbildunterstützung, bei dem
- mittels eines vorab im Navigationssystem kalibrierten Röntgengerätes eine Vielzahl von Röntgenbildern eines Patienten aus unterschiedlichen Positionen erstellt werden, während mittels eines medizinischen Navigationssystems eine Positionsbestimmung für das Röntgengerät erfolgt,
- die erhaltenen Patientendaten aus den erstellten Röntgenbildern durch Verarbeitung im Röntgengerät-System auf der Basis der Kalibrierung ausgewertet und räumlich umgesetzt werden,
- zusätzlich zu den ausgewerteten und umgesetzten Patientendaten noch die ursprünglichen zweidimensionalen Röntgenbilder und die den Röntgenbildern zugehörigen Erstellungsinformationen an das Navigationssystem übergeben werden, und bei dem
- die zweidimensionalen Röntgenbilder navigationsunterstützend auf einer Bildausgabe des Navigationssystems angezeigt werden.

2. Verfahren nach Anspruch 1, bei dem zur Kalibrierung des Röntgengeräts dessen Position in Relation zu einem vom Navigationssystem erfassbaren Kalibrierungsphantom ermittelt wird.

3. Verfahren nach Anspruch 1 oder 2, bei dem während der Kalibrierung des Röntgengeräts Transformationsmatrizen betreffend die Raumlage der aufgenommenen Röntgenbilder erstellt werden.

4. Verfahren nach Anspruch 3, bei dem mit der Übergabe der ursprünglichen, zweidimensionalen Röntgenbilder an das Navigationssystem auch die den einzelnen Röntgenbildern zugeordneten Transformationsmatrizen übergeben werden.

5. Verfahren nach einem der Ansprüche 1 bis 4, bei dem als Röntgengerät ein C-Bogen-Röntgengerät verwendet wird, das Röntgenbilder in verschiedenen Drehstellungen erstellt, insbesondere isozentrische Röntgenbildfolgen.

6. Programm gespeichert auf einem computer-lesbaren Speichermedium, das, wenn es auf einem Computer läuft oder in einem Computer geladen ist, den Computer veranlasst, ein Verfahren gemäß einem der Ansprüche 1 bis 5 durchzuführen.

## Claims

1. A method for displaying images in a medical navigation system assisted by x-ray images, wherein:
- a plurality of x-ray images of a patient are produced from different positions using an x-ray device which has been calibrated in the navigation system in advance, while the position of the x-ray device is determined using a medical navigation system;
- the patient data obtained from the x-ray images produced are evaluated and spatially converted by processing them in the x-ray device system based on the calibration;
- the original two-dimensional x-ray images and the production information corresponding to the x-ray images are transferred to the navigation system in addition to the evaluated and converted patient data; and wherein
- the two-dimensional x-ray images are displayed on an image output of the navigation system, to assist navigation.

2. The method as set forth in claim 1, wherein in order to calibrate the x-ray device, its position is ascertained in relation to a calibration phantom which can be detected by the navigation system.

3. The method as set forth in claim 1 or 2, wherein transformational matrices concerning the spatial position of the x-ray images recorded are produced while calibrating the x-ray device.

4. The method as set forth in claim 3, wherein the transformational matrices assigned to the individual x-ray images are also transferred to the navigation system when the original two-dimensional x-ray images are transferred.

5. The method as set forth in any one of claims 1 to 4, wherein a C-arc x-ray device is used as the x-ray device, said C-arc x-ray device producing x-ray images in various rotational positions, in particular in series of isocentric x-ray images.

6. A program stored on a computer-readable storage medium which, when running on a computer or loaded onto a computer, causes the computer to perform a method in accordance with any one of claims 1 to 5.

## Revendications

1. Procédé de visualisation pour un système de navigation pour applications médicales avec assistance d'images radiographiques pour lequel
- on réalise un grand nombre d'images radiographiques pour différentes positions d'un patient à l'aide d'un appareil à rayons X préalablement calibré dans le système de navigation, pendant qu'une détermination de position pour l'appareil à rayons X a lieu à l'aide d'un système de navigation pour applications médicales,
- les données de patient obtenues à partir des images radiographiques réalisées sont évaluées et transposées dans l'espace par traitement dans le système d'appareil à rayons X sur base du calibrage,
- en plus des données de patient évaluées et transposées, les images radiographiques bidimensionnelles d'origine et les informations d'établissement correspondant aux images radiographiques sont transmises au système de navigation, et pour lequel
- les images radiographiques bidimensionnelles sont affichées à titre d'assistance à la navigation sur un écran du système de navigation.

2. Procédé suivant la revendication 1, pour lequel on détermine pour le calibrage de l'appareil à rayons X la position de celui-ci par rapport à un modèle de calibrage pouvant être saisi par le système de navigation.

3. Procédé suivant la revendication 1 ou 2, pour lequel des matrices de transformation sont établies pendant le calibrage de l'appareil à rayons X en ce qui concerne la position spatiale des images radiographiques enregistrées.

4. Procédé suivant la revendication 3, pour lequel les matrices de transformation correspondant aux différentes images radiographiques sont également transmises au système de navigation en même temps que la transmission des images radiographiques bidimensionnelles d'origine.

5. Procédé suivant l'une des revendications 1 à 4, pour lequel on utilise comme appareil à rayons X un appareil à rayons X à courbure en C, qui réalise des images radiographiques dans différentes positions de rotation, en particulier des séquences d'images radiographiques isocentriques.

6. Programme mémorisé sur un support de stockage pouvant être lu par un ordinateur qui, lorsqu'il tourne sur un ordinateur ou est chargé dans un ordinateur, amène l'ordinateur à exécuter un procédé suivant l'une des revendications 1 à 5.
